# FASCICULE DE BREVET EUROPEEN

(11) **EP 0 625 706 B1**
(45) Date de publication et mention de la délivrance du brevet: **24.01.2001**
(21) Numéro de dépôt: 94430002.9
(22) Date de dépôt: 18.05.1994
(51) Int. Cl.: G01N 33/50

(54) **Méthode de protection des cellules leucocytaires, composition protectrice, composition de sang protégée, et méthode d'analyse sanguine**
Verfahren zum Schutz von Leukozyten, Schutzzusammensetzung, geschützte Blutzusammensetzung und Blutanalyseverfahren
Method to protect leucocytes, protective composition, protected blood composition and method to analyse blood

(30) Priorité: 19.05.1993 FR 9306332
(43) Date de publication de la demande: 23.11.1994
(73) Titulaire: Société Anonyme dite: IMMUNOTECH S.A., F-13276 Marseille Cédex 9 (FR)
(72) Inventeur: Van Agthoven, André, F-13009 Marseille (FR)
(74) Mandataire: Rinuy, Santarelli

(56) Documents cités:
- EP-A- 0 022 670
- EP-A- 0 161 770
- EP-A- 0 214 613
- EP-A- 0 530 490
- WO-A-85/05640

## Description

La présente invention concerne une méthode et une composition pour la protection des leucocytes notamment dans une procédure de lyse des érythrocytes, une composition de sang protégée et une méthode d'analyse sanguine.

Le sang contient plusieurs populations cellulaires. Les plus nombreuses sont les érythrocytes et les plaquettes fonctionnant dans l'échange et le transport de dioxyde de carbone et d'oxygène. Les plaquettes jouent un rôle dans la coagulation sanguine. Les leucocytes sont une population minoritaire impliquée dans le contrôle du système immunitaire.

Par analyse microscopique ou par analyse au cytomètre de flux, on distingue trois sous-populations dans les leucocytes : les cellules polynucléaires ayant plusieurs noyaux et les cellules mononucléaires avec un seul noyau, parmi lesquelles on peut distinguer les lymphocytes avec un noyau petit et rond et les monocytes avec un noyau de taille plus grande, réniforme.

Par cytométrie, en comparant la diffusion aux grands angles et la diffusion aux petits angles, on peut distinguer ces populations dans un cytogramme. La représentation de ces sous-populations leucocytaires est de 60 % environ pour les cellules polynucléaires, 30 % pour les lymphocytes et 10 % pour les monocytes. Des variations du pourcentage des sous-populations lymphocytaires peuvent donner une indication sur l'état de santé d'un individu.

Par marquage des cellules sanguines avec des anticorps monoclonaux conjugués à un marqueur de fluorescence et après analyse des cellules par microscopie, ou par cytométrie de flux, on peut distinguer les cellules sanguines en sous-populations encore plus finement qu'avec des moyens optiques simples.

Par exemple, avec le marqueur CD19, on peut distinguer dans la population des lymphocytes les cellules B qui sont dérivées de la moelle osseuse. Dans la même population, on peut distinguer, avec le marqueur CD3, les cellules T dérivées du thymus. Avec les marqueurs CD4 ET CD8, on peut distinguer respectivement les cellules T avec une fonction auxiliaire ou avec une fonction cytotoxique/suppresseur. L'identification des sous-classes des lymphocytes est importante pour le diagnostic ou le traitement des maladies du système immunitaire.
Du fait que les érythrocytes sont une population majoritaire dans le sang, ils peuvent masquer les leucocytes et rendre difficile leur analyse par cytométrie de flux.

Les techniques d'immunofluorescence conventionnelle incluent une séparation physique des cellules lymphocytes et des érythrocytes, par exemple par centrifugation en gradient de densité (Boyum, A.1968 Scand.J.Clin. Lab Invest., 21 Suppl 97).

Une autre méthode, plus rapide, est la lyse des érythrocytes dans le sang total. Par exemple, dans la méthode de Hansen (US-A-4.284.412 - EP-A- 0.022.670), un échantillon de sang traité avec un anti-coagulant est mélangé avec une préparation d'un anticorps conjugué fluorescent. Après incubation et lyse des érythrocytes, l'échantillon est passé dans un cytomètre de flux, afin d'analyser les populations leucocytaires positives pour l'anticorps en question.

Pour réaliser une analyse cytométrique correcte, il n'est pas seulement nécessaire de lyser tous les érythrocytes. Il faut aussi conserver tous les leucocytes dans un état morphologique où le cytomètre est capable de distinguer entre les cellules polynucléaires, les monocytes, les lymphocytes et le mélange de débris cellulaires et plaquettes. Il existe une grande diversité des méthodes de lyse érythrocytaire. Ces méthodes peuvent être basées, par exemple, sur un traitement acide, un traitement basique, un traitement à base de chlorure d'ammonium, d'alcool polyhydrique ou encore un choc hypotonique. Le problème de toutes ces méthodes est qu'en lysant les érythrocytes, des modifications sont introduites au niveau de la morphologie leucocytaire.

Dans les méthodes conventionnelles, on limite la dégradation leucocytaire, par exemple en procédant à l'analyse immédiatement après la lyse, par lavage des cellules, ou par l'addition d'un réactif de fixation comme le formaldéhyde ou le paraformaldéhyde.
Dans la méthode de Chang (C. Chang, US-A-4.902.613 -EP-A-0.161.770), la lyse est basée sur un traitement hypotonique avec un alcool polyhydrique. Durant cette lyse, les leucocytes sont stabilisés par le formaldéhyde et par un sel d'un acide faible. L'inconvénient de cette méthode est qu'en lysant et en fixant simultanément les cellules, il se forme des complexes de débris érythrocytaires et de plaquettes de taille significative qui interfèrent avec la population des lymphocytes dans le graphique de différenciation. De plus, la présence d'un alcool polyhydrique entraîne un déplacement des monocytes dans le graphique de différenciation qui, à leur tour, risquent d'interférer avec la population des lymphocytes. Un lavage après la lyse améliore le résultat du graphique de différenciation par l'enlèvement de débris érythrocytaires des plaquettes et par une renaturation des monocytes. L'inconvénient d'un lavage, en dehors du fait qu'il rallonge la procédure de préparation, est la perte de leucocytes. Avec la méthode décrite ci-dessus, on peut s'attendre à une perte de 20 à 40 % des leucocytes dans des échantillons normaux.
Cette perte s'explique par la force de centrifugation appliquée sur les cellules déjà partiellement dénaturées et l'interaction de certaines sous-populations cellulaires avec la paroi du tube ; elle peut conduire à une fausse numération cellulaire et peut prendre des proportions encore plus grandes quand il s'agit d'échantillons comprenant des leucocytes affaiblis par une maladie, par exemple le Syndrome d'Immuno Déficience Acquise (SIDA).

WO-A-85/05640 décrit un système de réactif et une méthode pour l'identification, la numération et l'examen de classes et de sous-classes de leucocytes sanguins, dans lequel on lyse les érythrocytes avec un réactif de lyse contenant de la saponine puis on traite les leucocytes avec un réactif de fixation bifonctionnel tel qu'un dialdéhyde.

EP-A- 0 530 490, décrit un système de réactif et une méthode pour le sous-typage des lymphocytes dans le sang périphérique, dans lequel on opère une fixation puis une lyse isotonique.

Il serait donc souhaitable de disposer d'un procédé d'analyses leucocytaires excluant tout lavage d'échantillons ainsi que la fausse numération des leucocytes.

C'est pourquoi la présente demande a pour objet une méthode et une composition pour protéger des cellules leucocytaires notamment de l'effet dénaturant d'une lyse érythrocytaire et pour inactiver des plaquettes afin de ne pas former des agrégats importants avec les débris érythrocytaires pendant la lyse, particulièrement la lyse par choc hypotonique simple. La méthode de protection selon la présente invention est assez efficace pour garantir une préparation d'échantillon stable avec une bonne séparation des sous-populations leucocytaires dans un graphique de différenciation obtenu par cytométrie de flux. Les propriétés fluorescentes des sous-populations obtenues par marquage préalable avec des anticorps conjugués sont tout à fait conservées durant cette procédure.

La présente demande a donc pour objet une méthode de protection des cellules leucocytaires, caractérisée en ce que l'on traite un échantillon de sang préalablement traité à l'aide d'un anticoagulant, à l'aide d'une composition sensiblement isotonique, ou hypertonique comprenant :
- un aldéhyde aliphatique,
- un sel d'un métal alcalin ou alcalino-terreux et d'un acide faible,
- si nécessaire, un agent d'isotonicité,
préalablement à une lyse hypotonique des érythrocytes, et plus généralement une méthode de protection des cellules leucocytaires, caractérisée en ce que l'on traite un échantillon de sang préalablement traité à l'aide d'un anticoagulant, à l'aide d'une composition de protection des leucocytes comportant un agent de fixation des leucocytes préalablement à une lyse hypotonique des érythrocytes.

La composition ci-dessus est pour simplifier la rédaction appelée ci-après "composition isotonique".

L'aldéhyde aliphatique en quantité suffisante conduit à une fixation de tous les composants cellulaires du sang. Cette fixation est obtenue par un pontage entre les groupes amino terminaux des protéines dans la paroi cellulaire. Des cellules ayant une grande densité de protéines dans la membrane de la surface cellulaire comme les leucocytes et les plaquettes sont davantage pontés, comparés aux érythrocytes ayant une densité plus faible. Ce traitement rend les cellules à grande densité protéique membranaire relativement résistantes à une procédure de lyse hypotonique subséquente.

Par "aldéhyde aliphatique", l'on entend de préférence ceux en C1-C4 ,par exemple l'acétaldéhyde, le butyraldéhyde, le glyoxal et notamment le formaldéhyde et le paraformaldéhyde.

La concentration de l'aldéhyde aliphatique dans le mélange de sang et de la composition isotonique va avantageusement de 0,036 M à 1,8 M et de préférence de 0,36 M à 1,8 M, notamment de 0,36 M à 0,72 M. Au-dessous de 0,36 M (0,1 % en poids par volume), la protection des leucocytes est insuffisante; au-dessus de 1,8 M, certains érythrocytes ne seront pas lysés.

Dans la présente demande, les valeurs d'aldéhyde aliphatique, notamment du formaldéhyde, sont indiquées pour un produit stabilisé à l'aide de 10 % de méthanol (0,84 M) tel celui commercialisé par la Société MERCK. Les quantités sont donc exprimées en aldéhyde brut.

D'autres agents de fixation des leucocytes sont par exemple des réactifs bifonctionnels comme le carbodiimide, le succinaldéhyde, ou le réactif de Mirsky.

Pour conserver la morphologie cellulaire durant la procédure de fixation de manière optimale, et pour donc réduire la taille des débris érythrocytaires, la composition isotonique selon l'invention contient aussi un sel d'un métal alcalin ou alcalino-terreux et d'un acide faible, tel que l'acide fumarique, malonique, succinique, citrique, pyruvique, lactique, aconitique, oxalique, ascorbique, phosphorique, carbonique, et de préférence l'acide formique, tartrique, acétique, notamment ce dernier, et de manière générale, les sels ci-dessus doués d'une bonne solubilité lorsque ce sont des sels de métaux alcalino-terreux.

Le métal alcalin ou alcalino-terreux est par exemple le lithium, le sodium, le potassium ou le magnésium, et de préférence ce dernier.

La concentration de l'acide faible du sel précité peut aller de 1mM à 1M dans le mélange de sang et de composition isotonique. En faible concentration de l'ordre de 1mM, cet acide faible améliore déjà la résolution des populations cellulaires au graphique de différenciation. Des concentrations supérieures à 1M d'acide faible interfèrent par contre avec la lyse hypotonique subséquente. Sa concentration va de préférence de 10 à 30 Mm et notamment de 15 à 20 Mm.

Par agent d'isotonicité, l'on entend un produit ou une composition destiné à être rajouté dans une quantité telle que la concentration finale de la composition est proche de la valeur physiologique, sans toutefois interférer sur la numération autrement que par l'isotonicité qu'il confère à la composition . L'agent d'isotonicité n'interfère notamment pas avec la lyse hypotonique subséquente et ne doit pas lyser les leucocytes. On peut citer par exemple des saccharides notamment des mono- ou di-saccharides comme le dextrose, le mannose, le glucose et particulièrement le saccharose.

La concentration de l'agent d'isotonicité devrait être telle qu'on ajuste la valeur isotonique du mélange pour protéger les cellules pendant la fixation, à une valeur sensiblement isotonique, ou hypertonique, de préférence peu hypertonique.

L'ajustement de ladite concentration dans la composition est du domaine de l'homme de l'art.

La lyse est faite ultérieurement à la mise en contact de l'échantillon avec la composition, par exemple de au moins une minute et de préférence au moins 5 minutes et tout particulièrement environ 10 minutes après la mise en contact.

Toutefois, compte tenu de la stabilité conférée par la composition selon l'invention, on peut procéder à la lyse jusqu'à 30 minutes après le traitement.

Dans des conditions préférentielles de mise en oeuvre de la méthode ci-dessus décrite, la composition isotonique renferme en outre une faible proportion d'un sel d'un cation divalent autre que celui du sel ci-dessus, notamment de manganèse ou de calcium lorsque le sel ci-dessus est un sel de magnésium. Celui-ci représente alors de préférence environ 5 à 15% de la concentration molaire du sel de métal alcalin ou alcalino-terreux et d'acide faible , et de préférence environ de 8%. Notamment, la concentration finale de calcium dans le mélange sang-composition isotonique est de 0,5 à 2,3 mM, de préférence de 1,1 à 1,6 mM.

Compte tenu de sa faible concentration, ce deuxième sel peut être un sel d'un acide faible, ou fort tel que l'acide chlorhydrique ou sulfurique.

La présente invention a également pour objet une composition pour la protection des leucocytes caractérisée en ce qu'elle comprend :
- un aldéhyde aliphatique choisi parmi l'acétaldéhyde, le butyraldéhyde, le formaldéhyde et le paraformaldéhyde,
- un sel d'un métal alcalin ou alcalino-terreux et d'un acide faible
- si nécessaire, un agent d'isotonicité, et en ce qu'elle est sensiblement isotonique, ou hypertonique.

Les compositions préférées sont celles définies dans les proportions et quantités ci-dessus. On retient tout particulièrement la composition qui est une solution aqueuse à environ 1,22 M de formaldéhyde, à environ 37 mM d'acétate de magnésium, à environ 3 mM de chlorure de calcium, et a un pH neutre de 7,0 environ. Cette composition renferme la quantité suffisante d'agent d'isotonicité ; par exemple, sa concentration de saccharose est environ 40 mM.

La présente demande a aussi pour objet une composition de sang protégée, caractérisée en ce qu'elle comprend un mélange d'un échantillon de sang traité par un anticoagulant, et d'une composition isotonique définie ci-dessus renfermant au moins 0,36 M d'aldéhyde aliphatique.

Une méthode de numération des leucocytes d'un échantillon sanguin mettant en oeuvre la méthode de protection selon l'invention est notamment la suivante, dans laquelle la lyse ultérieure est obtenue par choc hypotonique :
- Un échantillon de 0,1 ml de sang, traité avec un anticoagulant est mis en contact avec une préparation d'un ou deux anticorps conjugués fluorescents, de préférence dans un volume de 20 µl. Après incubation, le mélange est mis en contact avec la composition isotonique de protection. La composition isotonique est tout particulièrement une solution aqueuse à 1,22 M de formaldéhyde, à 37 mM d'acétate de magnésium, à 3 mM de chlorure de calcium, à 40 mM de saccharose et a un pH neutre, de 7,0 environ.
- Le volume de composition isotonique ci-dessus utilisé est 0,1 ml.

Après la mise en contact, le mélange est secoué fortement et laissé à température ambiante pendant une période de 10 minutes. Ensuite, une lyse érythrocytaire hypotonique est effectuée en rajoutant 1 ml d'eau distillée. La lyse est considérée comme totale au bout de 10 minutes à l'issue desquelles l'échantillon est prêt pour l'analyse cytométrique.

Toute la procédure est effectuée de préférence à température ambiante (18-24°C).
Avant l'analyse cytométrique, les échantillons peuvent être conservés pendant 6 heures à température ambiante ou pendant 48 heures à 4°C.
La méthode de lyse hypotonique de sang total a, selon les expériences de la demanderesse, une limitation vis-à-vis de l'appareil de cytométrie que l'on utilise. Les cytomètres de Becton Dickinson and Co., par exemple FACSCAN^{R} ou FACSTAR^{R}, sont bien adaptés à une lyse de ce genre. Des cytomètres d'autres types basés sur un système optique différent peuvent donner un résultat décevant ; ils sont plutôt adaptés à une lyse en conditions isotoniques.

C'est pourquoi la présente demande a aussi pour objet une méthode d'analyse de la population leucocytaire d'un échantillon sanguin dans laquelle l'on traite un échantillon sanguin à l'aide d'un anticoagulant, puis si désiré, à l'aide d'anticorps spécifiques d'au moins une population ou sous-population leucocytaire puis à l'aide d'un agent de fixation des leucocytes et ensuite procède à une lyse hypotonique puis à l'analyse par cytométrie de flux de l'échantillon, caractérisé en ce que après traitement à l'aide de l'anticoagulant et si désiré d'au moins un anticorps marqué, l'on traite l'échantillon à l'aide d'une composition sensiblement isotonique, ou hypertonique renfermant :
- un agent de fixation qui est un aldéhyde aliphatique,
- un sel d'un métal alcalin ou alcalino-terreux et d'un acide faible et
- si nécessaire, un agent d'isotonicité,
puis au moins 1 mn après traitement de l'échantillon à l'aide de la composition sensiblement isotonique ou hypertonique, l'on procède à une lyse hypotonique dudit échantillon.

La méthode ci-dessus peut notamment être mise en oeuvre dans les conditions préférentielles énoncées ci-dessus.

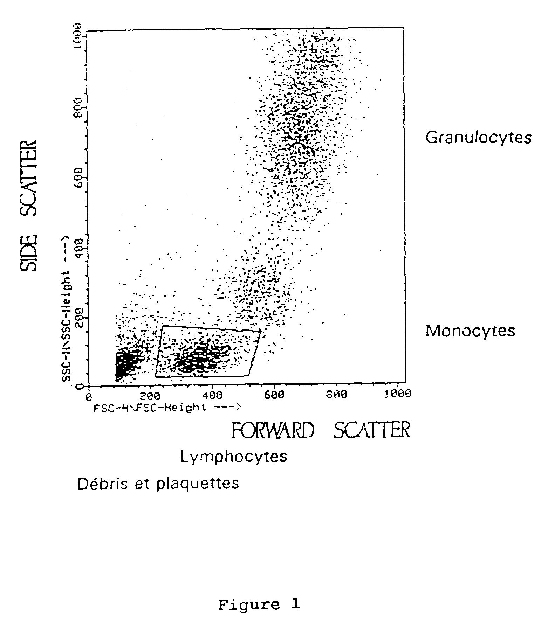

- La figure 1 montre un résultat de l'analyse de diffusion aux petits angles (forward scatter) et de diffusion aux grands angles (side scatter) d'un échantillon de sang traité à l'aide de la composition de l'exemple 2 et puis soumis à une lyse hypotonique, dans un cytogramme qui est obtenu en utilisant un cytomètre de flux "FACSCAN" commercialisé par Becton Dickinson and Co. De gauche à droite et de bas en haut, on remarque, respectivement, une bonne séparation des populations : plaquettes et débris érythrocytaires, lymphocytes, monocytes et polynucléaires. Pour étudier les lymphocytes, on peut introduire un seuil pour établir les débris et les plaquettes et mettre les lymphocytes dans un cadran tel que montré dans cette figure 1.
- Les figures 2 représentent une série d'analyses sur cytomètre FACSCAN de sang total en absence ou en présence des marqueurs fluorescents et préparés par réaction avec la composition de protection selon l'invention de l'exemple 2 et lyse hypotonique subséquente.

La figure 2a représente un cytogramme avec cadres dans les régions des lymphocytes (R₁), des monocytes (R₂) et des cellules polynucléaires (R₃).

La figure 2b représente un cytogramme de sang total en absence des marqueurs fluorescents.

La figure 2c représente un cytogramme de sang total en présence des marqueurs contrôles isotypiques. Diagramme sur le cadre R₁.

La figure 2d représente un cytogramme de sang total marqué avec CD45-FITC et CD14-PE. Diagramme sur le cadre R₁.

La figure 2e représente un cytogramme de sang total marqué avec CD45-FITC et CD14-PE. Cytogramme de FL2 des cellules dans le cadre monocytaire (R₂).

La figure 2f représente un cytogramme de sang total marqué avec CD3-FITC et CD4-PE.

La figure 2g représente un cytogramme de sang total marqué avec CD3-FITC et CD8-PE.

La figure 2h représente un cytogramme de sang total marqué avec CD3-FITC et CD19-PE.
Les exemples qui suivent illustrent la présente invention sans toutefois la limiter.

### EXEMPLE 1 : Composition isotonique

On a préparé une composition isotonique aqueuse selon l'invention et ayant la composition suivante :
- formaldéhyde* 0,206 M
- acétate de magnésium 0,037 M
- chlorure de calcium 0,03 M
- saccharose 0,04 M
- eau distillée
pH = 7,0
* formaldéhyde stabilisé par 10 % de méthanol

### EXEMPLE 2 : Composition isotonique

On a préparé une composition isotonique aqueuse selon l'invention et ayant la composition suivante :
- formaldéhyde* 0,1 M
- tartrate double de potassium et de sodium 0,060 M
- glucose 0,1 M
- eau distillée
pH = 7,0
* formaldéhyde stabilisé par 10 % de méthanol

### EXEMPLE 3 : Analyse d'un échantillon sanguin

### A) Des aliquotes de 0,1 ml de sang sont réparties dans six tubes.

Dans le tube 1, on ajoute 20 µl de PBS (sel physiologique tamponné au phosphate).

Dans le tube 2, 20 µl d'un mélange de deux anticorps monoclonaux (contrôles isotypiques) avec une spécificité irrelevante, à savoir spécifique d'un composé n'existant pas naturellement chez l'homme, l'un conjugué à la fluorescéine isothiocyanate (FITC): 10 µl, l'autre à la phycoérythrine (PE): 2 µl, PBS (8 µl). commercialisés sous les référence de la gamme IOTEST 0639 et 0670 respectivement.

Dans le tube 3, 20 µl d'un mélange d'anticorps monoclonal 0782 de la gamme IOTEST avec une spécificité CD45 conjugué à la FITC (2 µl) et d'anticorps monoclonal clone RM052 0650 de la gamme IOTEST (2,5 µl) avec une spécificité CD14 conjugué à la phycoérythrine, PBS 15,5 µl.

Dans le tube 4, 20 µl d'un mélange d'anticorps UCHTl référence 1281 avec une spécificité CD3 conjugué FITC (2 µl), et d'anticorps 13B8.2 référence 0449 avec une spécificité CD4 conjugué PE (18 µl).

Dans le tube 5, 20 µl d'un mélange d'anticorps UCHTl référence 1281 (CD3-FITC): 2 µl et d'anticorps B9.11 (CD8-PE) référence 0452 (33 µl) PBS (16,7 µl).

Dans le tube 6, 20 µl d'un mélange d'anticorps UCHTl référence 1281 (CD3-FITC): 2 µl et d'anticorps J4.119 (CD19-PE) référence 1285 (10 µl), PBS 8µl.

Tous les anticorps ci-dessus sont commercialisés par la Société IMMUNOTECH (FRANCE) dans leur gamme IOTEST.

Après incubation de 20 minutes avec les anticorps, 0,1 ml de composition isotonique de l'exemple 1 est ajouté et les tubes sont secoués.

Après 10 minutes de réaction, un volume de 1 ml d'eau distillée est rajouté et les tubes secoués.

Après trois heures de conservation à température ambiante, les échantillons sont analysés en cytométrie de flux par un cytomètre FACSCAN de Becton Dickinson and Co. Dans le cytogramme, un seuil est placé à 200 unités sur l'échelle des petits angles, afin d'éviter la numération des débris et des plaquettes. Un cadre est mis autour des populations de lymphocytes, monocytes et cellules polynucléaires. Une analyse en fluorescence 1 (528 nm) détectant les conjugués FITC et en fluorescence 2 (545 nm) détectant les conjugués PE, est effectuée sur les cellules lymphocytaires encadrées.

### B) Interprétation :

Les cellules positives en fluorescence pour CD45 représentent tous les leucocytes en permettant de distinguer les leucocytes et les débris plus plaquettes.

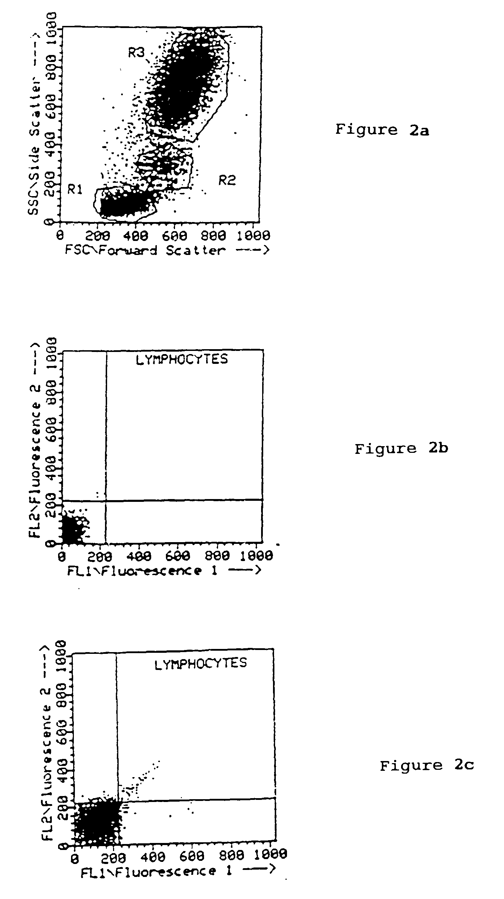

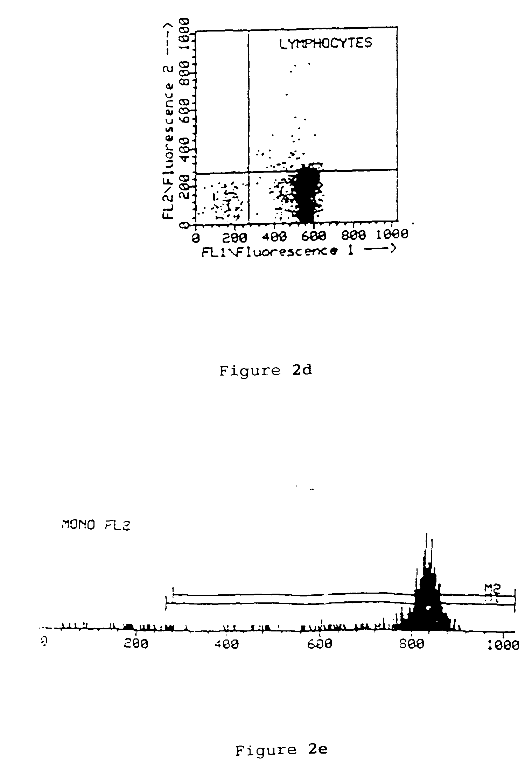

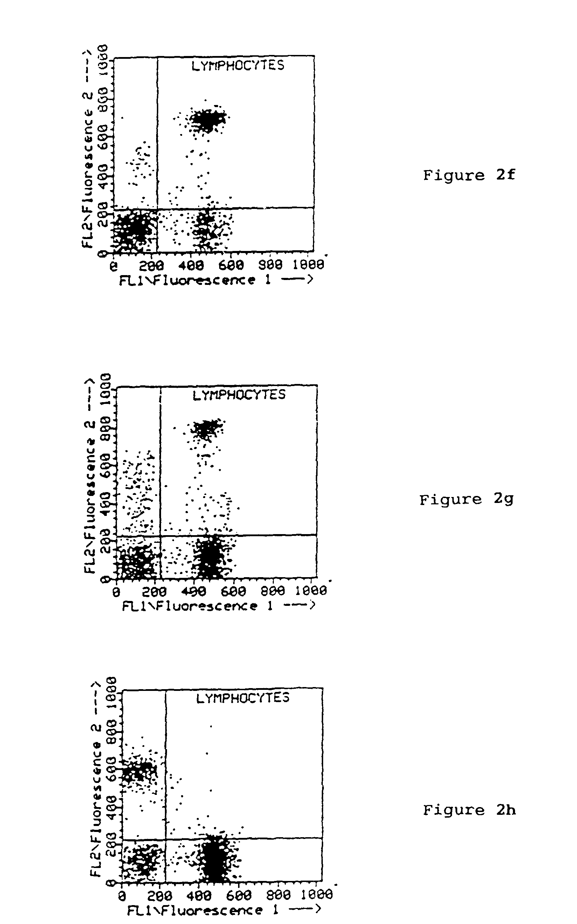

Les cellules positives pour CD14 sont des monocytes permettant de distinguer entre monocytes et lymphocytes, et des cellules positives pour CD3 et CD4, les cellules T auxiliaires.
Les cellules positives pour CD3 et CD8 indiquent les cellules T cytotoxiques/suppresseur, les cellules positives pour CD3, les cellules T et les cellules positives pour CD19, les cellules B.
- Les résultats sont représentés sur les figures 2.

On observe que dans le cadre lymphocytaire, 97 % des événements sont de vrais lymphocytes, 68 % sont des cellules T, 17 % sont des cellules B et 15 % sont ni T, ni B.

Dans la population T, 53 % sont des cellules auxiliaires et 16 % sont des cellules cytotoxiques/suppressives.

Dans le cadre monocytaire, 96 % des événements sont de vrais monocytes.

### CONCLUSION :

On conclut de l'analyse de la figure 1 que la séparation des populations leucocytaires et débris est bien réalisée dans le cytogramme.

La figure 2 montre que la méthode selon la présente invention sauvegarde les propriétés fluorescentes des cellules marquées et que l'on peut effectuer une bonne discrimination cellulaire du sang total.

La comparaison effectuée avec des déterminations effectuées en parallèle avec la méthode FACSLYSE de Becton-Dickinson and Co. montre que l'on obtient avec la présente invention des valeurs sensiblement identiques.

### EXEMPLE 4 : Composition

On a préparé une composition aqueuse selon l'invention et ayant la composition suivante :
- formaldéhyde * 0,185 M
- polyphosphate de sodium 1 % Poids/Volume
- saccharose 20 mM
- carbonate de sodium 10 mM
- eau ditillée
pH = 7,0
* formaldéhyde stabilisé par 10 % de méthanol

## Revendications

1. Méthode de protection des cellules leucocytaires, caractérisée en ce que l'on traite un échantillon de sang préalablement traité à l'aide d'un anticoagulant, à l'aide d'une composition de protection des leucocytes comportant un agent de fixation des leucocytes préalablement à une lyse hypotonique des érythrocytes.

2. Méthode de protection des cellules leucocytaires, caractérisée en ce que l'on traite un échantillon de sang préalablement traité à l'aide d'un anticoagulant, à l'aide d'une composition sensiblement isotonique, ou hypertonique, comprenant :
- un aldéhyde aliphatique,
- un sel d'un métal alcalin ou alcalino-terreux et d'un acide faible
- si nécessaire, un agent d'isotonicité,
préalablement à une lyse hypotonique des érythrocytes.

3. Méthode selon la revendication 1 ou 2, caractérisée en ce que l'échantillon est traité avec un anticorps spécifique d'au moins une sous-population lymphocytaire préalablement au traitement à l'aide de la composition de protection.

4. Méthode selon l'une des revendications 1 à 3, caractérisée en ce que la lyse est effectuée au moins 1 minute après mise en contact de l'échantillon avec la composition de protection.

5. Composition pour la protection des leucocytes dans un échantillon de sang traité par un anticoagulant, caractérisée en ce qu'elle comprend :
- un aldéhyde aliphatique choisi parmi l'acétaldéhyde, le butyraldéhyde, le formaldéhyde et le paraformaldéhyde,
- un sel de métal alcalin ou alcalino-terreux et d'un acide faible
- si nécessaire un agent d'isotonicité
et en ce qu'elle est sensiblement isotonique, ou hypertonique.

6. Composition selon la revendication 5, caractérisée en ce que l'aldéhyde aliphatique est le formaldéhyde ou le paraformaldéhyde.

7. Composition selon l'une des revendications 5 ou 6, caractérisée en ce que le sel de métal alcalin ou alcalino-terreux d'un acide faible est un sel de sodium, potassium, ou magnésium, de l'acide acétique, tartrique ou formique.

8. Composition selon l'une des revendications 5 à 7, caractérisée en ce qu'elle renferme en outre un second sel qui est un sel de manganèse ou de calcium.

9. Composition de sang protégée, caractérisée en ce qu'elle comprend un mélange d'un échantillon de sang traité par un anticoagulant, et d'une composition selon l'une des revendications 5 à 8 renfermant au moins 0,36 M d'aldéhyde aliphatique.

10. Méthode d'analyse de la population leucocytaire d'un échantillon sanguin dans laquelle l'on traite un échantillon sanguin à l'aide d'un anticoagulant, puis si désiré, à l'aide d'anticorps spécifiques d'au moins une population ou sous-population leucocytaire puis à l'aide d'un agent de fixation des leucocytes et ensuite procède à une lyse hypotonique puis à l'analyse par cytométrie de flux de l'échantillon, caractérisé en ce que après traitement à l'aide de l'anticoagulant et si désiré d'au moins un anticorps marqué, l'on traite l'échantillon à l'aide d'une composition sensiblement isotonique, ou hypertonique renfermant :
- un agent de fixation qui est un aldéhyde aliphatique,
- un sel d'un métal alcalin ou alcalino-terreux et d'un acide faible et
- si nécessaire, un agent d'isotonicité,
puis au moins 1 mn après traitement de l'échantillon à l'aide de la composition sensiblement isotonique ou hypertonique, l'on procède à une lyse hypotonique dudit échantillon.

## Patentansprüche

1. Verfahren zum Schutz von Leukozyten, dadurch gekennzeichnet, dass eine zuvor mit einem Antikoagulans behandelte Blutprobe vor einer hypotonischen Lyse der Erythrozyten mit einer Zusammensetzung zum Schutz von Leukozyten, umfassend ein Agens zur Fixierung der Leukozyten, behandelt wird.

2. Verfahren zum Schutz von Leukozyten, dadurch gekennzeichnet, dass eine zuvor mit einem Antikoagulans behandelte Blutprobe vor einer hypotonischen Lyse der Erythrozyten mit einer im Wesentlichen isotonischen oder hypertonischen Zusammensetzung, umfassend:
- einen aliphatischen Aldehyd,
- ein Salz eines alkalischen oder erdalkalischen Metalls und einer schwachen Säure,
- wenn notwendig ein isotonisches Agens,
behandelt wird.

3. Verfahren nach Anspruch 1 oder 2, dadurch gekennzeichnet, dass die Probe vor der Behandlung mit der Schutzzusammensetzung mit einem für zumindest eine Lymphozytenuntergruppe spezifischen Antikörper behandelt wird.

4. Verfahren nach einem der Ansprüche 1 bis 3, dadurch gekennzeichnet, dass die Lyse zumindest 1 Minute nach dem In-Kontakt-Bringen der Probe mit der Schutzzusammensetzung durchgeführt wird.

5. Zusammensetzung zum Schutz der Leukozyten in einer mit einem Antikoagulans behandelten Blutprobe, dadurch gekennzeichnet, dass sie umfasst:
- einen aliphatischen Aldehyd, ausgewählt aus Acetaldehyd, Butyraldehyd, Formaldehyd und Paraformaldehyd,
- ein Salz eines alkalischen oder erdalkalischen Metalls und einer schwachen Säure,
- wenn notwendig ein isotonisches Agens
und dadurch, dass sie im Wesentlichen isotonisch oder hypertonisch ist.

6. Zusammensetzung nach Anspruch 5, dadurch gekennzeichnet, dass der aliphatische Aldehyd Formaldehyd oder Paraformaldehyd ist.

7. Zusammensetzung nach einem der Ansprüche 5 oder 6, dadurch gekennzeichnet, dass das Salz des alkalischen oder erdalkalischen Metalls einer schwachen Säure ein Natrium-, Kalium- oder Magnesiumsalz der Essig-, Wein- oder Ameisensäure ist.

8. Zusammensetzung nach einem der Ansprüche 5 bis 7, dadurch gekennzeichnet, dass sie weiters ein zweites Salz umfasst, das ein Mangan- oder Calciumsalz ist.

9. Geschützte Blutzusammensetzung, dadurch gekennzeichnet, dass sie ein Gemisch einer mit einem Antikoagulans behandelten Blutprobe und einer Zusammensetzung nach einem der Ansprüche 5 bis 8, umfassend zumindest 0,36 M eines aliphatischen Aldehyds, umfasst.

10. Verfahren zur Analyse einer Leukozytengruppe einer Blutprobe, wobei eine Blutprobe mit einem Antikoagulans, anschließend, wenn gewünscht, mit für zumindest eine Leukozytengruppe oder -untergruppe spezifischen Antikörpern, anschließend mit einem Agens zur Fixierung der Leukozyten behandelt wird und schließlich eine hypotonische Lyse, dann die zytometrische Fließanalyse der Probe durchgeführt wird, dadurch gekennzeichnet, dass nach Behandlung mit dem Antikoagulans, und wenn gewünscht zumindest einem markierten Antikörper, die Probe mit einer im Wesentlichen isotonischen oder hypertonischen Zusammensetzung, umfassend:
- ein Agens zur Fixierung, das ein alipathischer Aldehyd ist,
- ein Salz eines alkalischen oder erdalkalischen Metalls und einer schwachen Säure und
- wenn notwendig ein isotonisches Agens,
behandelt wird, anschließend zumindest 1 Minute nach der Behandlung der Probe mit Hilfe der im Wesentlichen isotonischen oder hypertonischen Zusammensetzung eine hypotonische Lyse der Probe durchgeführt wird.

## Claims

1. A method for protecting leucocyte cells, characterized in that a blood sample which has been pre-treated with an anticoagulant is treated using a leucocyte protective composition comprising a leucocyte fixative prior to a hypotonic lysis of the erythrocytes.

2. A method for protecting leucocyte cells, characterized in that a blood sample which has been pre-treated with an anticoagulant, is treated using a substantially isotonic or hypertonic composition comprising:
- an aliphatic aldehyde,
- a salt of an alkali metal or an alkaline earth metal and a weak acid,
- if necessary, an agent to render the composition isotonic.

3. A method according to claim 1 or 2, characterized in that the sample is treated with an antibody specific for at least one lymphocyte subpopulation before the treatment with the protective preparation

4. A method according to one of claims 1 to 3, characterized in that the lysis is carried out at least one minute after the sample is brought into contact with the protective preparation.

5. Composition for the protection of leucocytes in a blood sample treated with an anticoagulant, characterized in that it comprises:
- an aliphatic aldehyde chosen from acetaldehyde, butyraldehyde, formaldehyde and paraformaldehyde,
- a salt of an alkali metal or an alkaline earth metal and a weak acid,
- if necessary, an agent to render the composition isotonic
and in that it is substantially isotonic or hypertonic.

6. Composition according claim 5, characterized in that the aliphatic aldehyde is formaldehyde or paraformaldehyde.

7. Composition according one of claims 5 or 6, characterized in that the alkali metal or alkaline earth metal salt of a weak acid is a sodium, potassium or magnesium salt of acetic, tartaric or formic acid.

8. Composition according one of claims 5 to 7, characterized in that in addition it contains a second salt which is a manganese or calcium salt.

9. Composition of protected blood, characterized in that it comprises a mixture of a blood sample treated with an anticoagulant and a composition according to one of claims 5 to 8 containing at least 0.36 M of aliphatic aldehyde.

10. A method for analyzing the leucocyte population of a blood sample in which the blood sample is treated with an anticoagulant, then if desired, with antibodies specific to at least one leucocyte population or sub-population; then with a leucocyte fixative and then a hypotonic lysis is carried out then the sample is analyzed by flow cytometry, characterized in that after treatment with the anticoagulant and if desired at least one labelled antibody, the sample is treated with a substantially isotonic or hypertonic composition containing:
a fixative which is an aliphatic aldehyde,
a salt of an alkali metal or an alkaline earth metal and a weak acid,
if necessary, an agent to render the composition isotonic,
then at least one minute after treatment of the sample with the substantially isotonic or hypertonic composition, said sample is lyzed under hypotonic conditions.
